(19) Europäisches Patentamt
European Patent Office
Office européen des brevets

(11) **EP 1 980 292 A2**

(12) **EUROPEAN PATENT APPLICATION**

(43) Date of publication:
**15.10.2008 Bulletin 2008/42**

(51) Int Cl.:
**A61N 1/365** (2006.01)   **A61B 5/11** (2006.01)
**A61B 7/00** (2006.01)   **A61B 5/0215** (2006.01)

(21) Application number: **08251415.9**

(22) Date of filing: **11.04.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **11.04.2007 US 734137**

(71) Applicant: **PACESETTER, INC.**
**Sylmar, CA 91392-9221 (US)**

(72) Inventor: **Schecter, Stuart O.**
**Great Neck,**
**NY 11021 (US)**

(74) Representative: **Phillips, Emily Elizabeth et al**
**Kilburn & Strode**
**20 Red Lion Street**
**London WC1R 4PJ (GB)**

(54) **Pulmonary pressure monitoring**

(57)     Devices, methods, and systems for determining a systolic pulmonary artery pressure index (PAPi) corresponding to pulmonary artery pressure (PAP) and/or right ventricular systolic pressure (RVSP) use lead-based electronic sensors detecting right heart valvular events. Suitable sensors include impedance sensors, accelerometers, cardiomechanical electric sensors, and sonomicrometers.

FIG. 1A

EP 1 980 292 A2

**Description**

Background

The present disclosure relates to cardiac monitoring, and more particularly, to determining a pulmonary artery pressure index.

Pulmonary hypertension (PHT) is often a consequence of end-stage cardiomyopathy and valvular heart disease. Evolving device-based technologies using transthoracic impedance data are capable of monitoring pulmonary/thoracic fluid content, which indicates changes in clinical status due to progressive congestive heart failure. Patients with PHT may be relatively protected from developing increases in pulmonary congestion (increases in thoracic fluid content) and may present, clinically decompensated, with right-sided heart failure without significant decreases in thoracic impedance. Such patients may also be susceptible to develop acute pulmonary edema with high risk of mortality.

In the chronic state, fluid builds up in the liver (passive liver congestion) and the lower extremities (peripheral edema). For a variety of reasons, the pulmonary parenchyma may be spared from extravasation of fluid secondary to physiologic changes in the pulmonary vascular bed (e.g., arteriolar thickening secondary to pulmonary hypertension). Clinical deterioration may go unnoticed, until irreversible, end-organ damage occurs and/or acute pulmonary edema occurs precipitously with little time for intervention. For these reasons, pure thoracic impedance monitoring can have decreased sensitivity and/or specificity for detection of progressive heart failure.

Systolic pulmonary artery pressure (PAP) and/or right ventricular systolic pressure (RVSP) are indicators of PHT. Consequently, detecting changes in right heart pressure can identify at risk patients earlier. PAP and RVSP can be measured using tissue Doppler echocardiography of the free wall of the right ventricle (RV); however, such measurements are typically taken in a clinical setting, and are, accordingly, inconvenient for patients.

Devices, methods, and systems for determining a systolic pulmonary artery pressure index (PAPi) corresponding to pulmonary artery pressure (PAP) and/or right ventricular systolic pressure (RVSP) use lead-based electronic sensors detecting right heart valvular events. Suitable sensors include impedance sensors, accelerometers, cardiomechanical electric sensors, and sonomicrometers.

Some embodiments provide an implantable cardiac stimulation device comprising: at least one lead adapted to be implanted within the right ventricle of the heart, wherein the at least one lead is adapted to provide therapeutic stimulation to the heart of the patient; at least one valve sensor wherein the sensor is adapted to be implanted within a chamber of the heart wherein the at least one sensor is further adapted to sense valvular events within the right ventricle of the heart and provide valvular event signals indicative thereof; a controller that induces the delivery of therapeutic stimulation to the heart of the patient via the at least one lead and receives the valvular event signals from the at least one valve sensor, wherein the controller uses the valvular event signals to periodically determine a time parameter at least partially representative of the time period between the closing of the pulmonary valve and the opening of the tricuspid valve of the right ventricle of the heart and wherein the controller assess whether the periodically determined time parameter is indicative of increasing pulmonary artery pressure and wherein the controller stores data indicative of the increasing pulmonary artery pressure for subsequent downloading.

In some embodiments, the at least one valve sensor is a pressure sensor that senses a signal indicative of the movement of the walls of the right ventricle. In some embodiments, the pressure sensor is a CMES sensor. In some embodiments, the at least one valve sensor comprises an impedance sensor that senses the impedance within the right ventricle to determine the occurrence of the valvular events. In some embodiments, the at least one sensor comprises an accelerometer.

In some embodiments, the controller assess whether the periodically determined time parameter is indicative of increasing pulmonary artery pressure by normalizing the time characteristic.

In some embodiments, at least one lead also provides an intra-cardiac electrogram signal to the controller and wherein the time parameter is normalized by dividing the time characteristic by the square root of a measured time period for a cycle of the intra-cardiac electrogram signal so as to correct the time parameter for heart rate.

In some embodiments, the controller uses the normalized time parameter to periodically assess whether the normalized time parameter is potentially indicative of increasing pulmonary artery pressure. In some embodiments, the controller determines whether the periodically determined time parameter is potentially indicative of increasing pulmonary artery pressure by periodically averaging a first time period set of normalized time parameter values and a second time period set of normalized time parameter values wherein the second time period a longer duration than the first time period and then determining a ratio between the first time period set of normalized time parameter values and the second time period set of normalized time parameter values. In some embodiments, the first time period is from about 1 to about 7 days and the second time period is from about 1 to about 6 months.

Some embodiments provide a method for determining a pulmonary artery pressure index comprising: positioning a lead comprising a sensor system at a right ventricle; acquiring with the sensor system data encoding right heart valvular timing, wherein the data does not comprise direct pressure measurement; extracting from the data encoding right heart valvular timing a pulmonary valve closure and a tricuspid valve opening; determining an isovolumetric relaxation time from the pulmonary valve closure and the tricuspid valve opening; and calculating a pulmonary artery pressure index from the isovolumetric relaxation time.

In some embodiments, the sensor system comprises a plurality of right ventricular electrodes for acquiring real-time impedance waveforms. In some embodiments, the sensor system comprises an acoustic sensor for acquiring valvular heart sounds. In some embodiments, the acoustic sensor comprises a sonomicrometer. In some embodiments, the acoustic sensor comprises a cardiomechanical electric sensor. In some embodiments, the sensor system comprises an accelerometer for acquiring myocardial motion. In some embodiments, the myocardial motion comprises a right-ventricle free-wall motion.

Some embodiments further comprise ensemble averaging the data acquired by the sensor system to improve the signal to noise ratio. Some embodiments further comprise applying a band-pass filter to the data acquired by the sensor system to improve the signal to noise ratio.

In some embodiments, at least a portion of the data is acquired over a period of relative apnea. In some embodiments, the period of relative apnea is detected using an implanted respirometer. In some embodiments, at least a portion of the data is acquired during a period of rest.

Some embodiments further comprise monitoring pulmonary artery pressure index over time to detect acute changes.

Some embodiments provide an implantable device for determining a pulmonary artery pressure index comprising: an implantable sensor system comprising at least one of an accelerometer, a cardiomechanical electric sensor, and a sonomicrometer disposed on a lead, wherein the sensor system is operable to acquire data encoding right heart valvular timing and output a corresponding signal; and an implantable controller system coupled to the sensor system operable to convert the signal from the sensor system into a pulmonary artery pressure index.

Some embodiments provide a device for measuring a pulmonary artery pressure index comprising: an implantable lead comprising a sensor system operable to detect right-heart valvular timing and output a corresponding signal, wherein the sensor system does not comprise an electrical sensor or a direct pressure measuring sensor; and an implantable controller system coupled to the sensor system operable to convert the signal from the sensor system into a pulmonary artery pressure.

In some embodiments, the controller system is disposed in a case. Some embodiments further comprise an accelerometer disposed in the case. Some embodiments further comprise a respirometer coupled to the controller system.

Some embodiments further comprise a left atrial lead and a coronary sinus lead, both of which are coupled to the controller system, wherein the implantable lead is a right ventricular lead, and the controller is operable to treat cardiac arrhythmia with stimulation therapy.

Some embodiments provide a system for measuring a pulmonary artery pressure index comprising: an implantable lead comprising a sensor system operable to detect right-heart valvular timing and output a corresponding signal, wherein the sensor system does not comprise an electrical sensor or a direct pressure measuring sensor; and an implantable controller system coupled to the sensor system operable to convert the signal from the sensor system into a pulmonary artery pressure by a method comprising: contacting the lead comprising the sensor system with a right ventricle; detecting with the sensor system a pulmonary valve closure and a tricuspid valve opening; determining an isovolumetric relaxation time from the pulmonary valve closure and the tricuspid valve opening; and calculating a pulmonary artery pressure index from the isovolumetric relaxation time.

Brief Description Of The Drawings

FIGS. 1A, 1B, and 1C illustrate embodiments of a device for determining a pulmonary artery pressure index.

FIG. 2 illustrates a cross section of an embodiment of a cardiomechanical electric sensor.

FIG. 3 is a block diagram of Figures 1A-2 as shown above.

FIG. 4 is a flow diagram illustrating an embodiment of a method for determining a pulmonary artery pressure index.

FIG. 5A illustrates a normal echocardiogram waveform suitable for quantifying peak blood cell velocities within a TR jet. FIG. 5B illustrates echocardiogram exhibiting inadequate regurgitant tricuspid blood flow to permit quantifying peak blood cell velocities.

FIG. 6 illustrates an echocardiogram of a tricuspid valve with poor coaptation.

FIG. 7 illustrates a myocardial velocity-time graph of a region of interest (ROI) on an RV free wall.

FIG. 8 compares waveforms generated by surface ECG and cardiomechanical sensor (CMES) measurements.

FIG. 9 compares results of ECG, CMES, and tissue Doppler measurements.

FIG. 10 overlays the tissue-Doppler-measured velocity-time curves of an RV lead (RV1) and a region of interest on the RV free wall (RVfw).

FIG. 11 illustrates an example of a time lag t in a CMES measurement. Detailed Description

Increases in pulmonary pressure may occur as a result of a variety of pathophysiologic processes, including causes such as left-sided cardiac failure, primary pulmonary hypertension, chronic obstructive pulmonary disease and post-cardiac transplantation. Thus, pulmonary pressure is a sensitive indicator and, in many ways, a common denominator of cardiac performance, pulmonary function, and valvular pathology in addition to pulmonary vascular congestion. Monitoring pulmonary pressure over time allows a clinician to detect progressive heart failure and identify changes secondary to valvular heart disease before such changes become irreversible, thereby providing more comprehensive insight into the pathophysiologic state. Incorporating pulmonary pressure measurement in an implanted cardiac rhythm management (CRM) device permits continuous, long term monitoring of an at-risk population. An implantable cardiac rhythm management (CRM) device with right-heart pressure monitoring capabilities is a valuable diagnostic tool for assessing combinations of cardiac performance, valvular function, pulmonary pressure, and pulmonary vascular congestion. Those skilled in the art will understand that other devices are also useful for monitoring right-heart pressure.

Reference will now be made to the drawings wherein like numerals refer to like parts throughout. This description is not to be taken in a limiting sense but is made merely for the purpose of describing certain general principles. All references cited herein are incorporated by reference in their entireties.

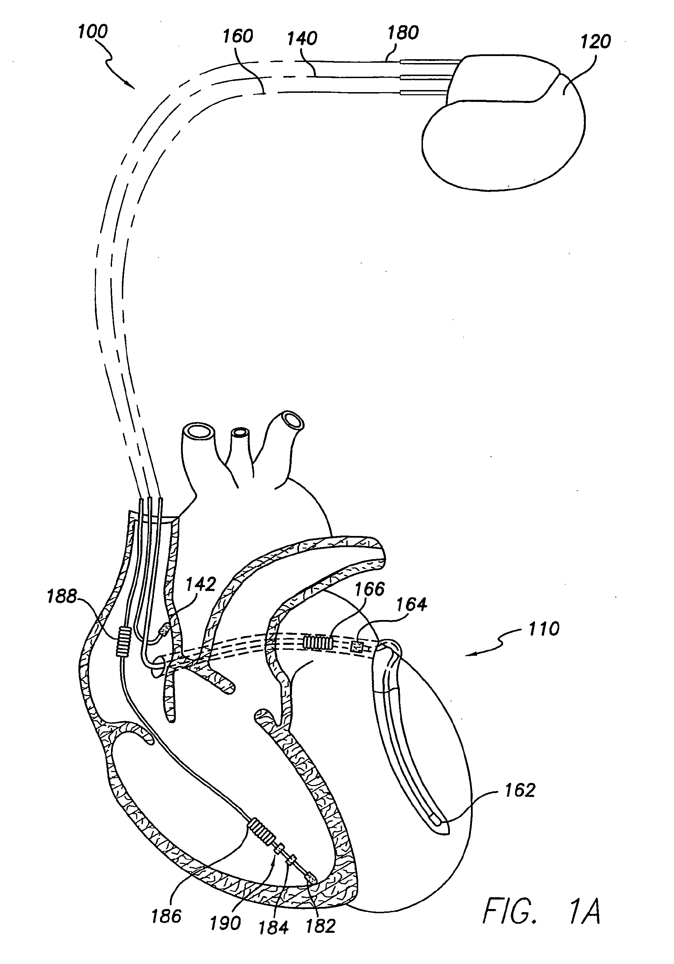

An embodiment of a suitable device is illustrated in FIG. 1A. In this embodiment, the stimulation device 100 is in electrical communication with a patient's heart 110 by way of three leads, 140, 160, and 180, suitable for delivering multi-chamber stimulation and shock therapy. To sense atrial cardiac signals and to provide right atrial chamber stimulation therapy, the stimulation device 100 is coupled to an implantable right atrial lead 140 having at least an atrial tip electrode 142, which typically is implanted in the patient's right atrial appendage.

To sense left atrial and ventricular cardiac signals and to provide left chamber pacing therapy, the stimulation device 100 is coupled to a coronary sinus lead 160 designed for placement in the coronary sinus region via the coronary sinus ostium (OS) for positioning a distal electrode adjacent to the left ventricle and/or additional electrode(s) adjacent to the left atrium. As used herein, the phrase "coronary sinus region" refers to the vasculature of the left ventricle, including any portion of the coronary sinus, great cardiac vein, left marginal vein, left posterior ventricular vein, middle cardiac vein, and/or small cardiac vein or any other cardiac vein accessible by the coronary sinus.

Accordingly, an exemplary coronary sinus lead 160 is designed to receive atrial and ventricular cardiac signals and to deliver left ventricular pacing therapy using at least a left ventricular tip electrode 162, left atrial pacing therapy using at least a left atrial ring electrode 164, and shocking therapy using at least a left atrial coil electrode 166.

The stimulation device 100 is also shown in electrical communication with the patient's heart 110 by way of an implantable right ventricular lead 180 having, in this embodiment, a right ventricular tip electrode 182, a right ventricular ring electrode 184, a right ventricular (RV) coil electrode 186, and a superior vena cava (SVC) coil electrode 188. Typically, the right ventricular lead 180 is transvenously inserted into the heart 110 so as to place the right ventricular tip electrode 182 in the right ventricular apex so that the RV coil electrode 186 is positioned in the right ventricle and the SVC coil electrode 188 is positioned in the superior vena cava. Accordingly, the right ventricular lead 180 is capable of receiving cardiac signals, and delivering stimulation in the form of pacing and shock therapy to the right ventricle.

The device 100 also comprises a sensor system 190 comprising one or more sensors operable to acquire data indicative of and/or encoding right heart valvular timing. In preferred embodiments, sensors in the sensor system 190 are disposed on one or more leads, for example, the right ventricular lead 180. The lead-based nature of the sensor system 190 simplifies implantation by reducing the number of components to be implanted, as well as reducing possible undesirable interactions between multiple components within the RV.

Preferably, the sensor system 190 does not detect pressure directly. In the illustrated embodiment, the sensor system 190 comprises the tip electrode 182 and the ring electrode 184, which detect cardiac electrical activity inductively. Other embodiments do not use electrical detection to determine right heart valvular timing. For example, in some embodiments, the sensor system 190 comprises accelerometers for detecting myocardial motion. In some embodiments, the sensor system 190 comprises piezoelectric or cardiomechanical electric sensors (CMES), for example, as described in U.S. Patent No. 6,886,411. Embodiments of CMES are operable to detect valvular acoustics and/or myocardial motion, for example, RV free wall motion.

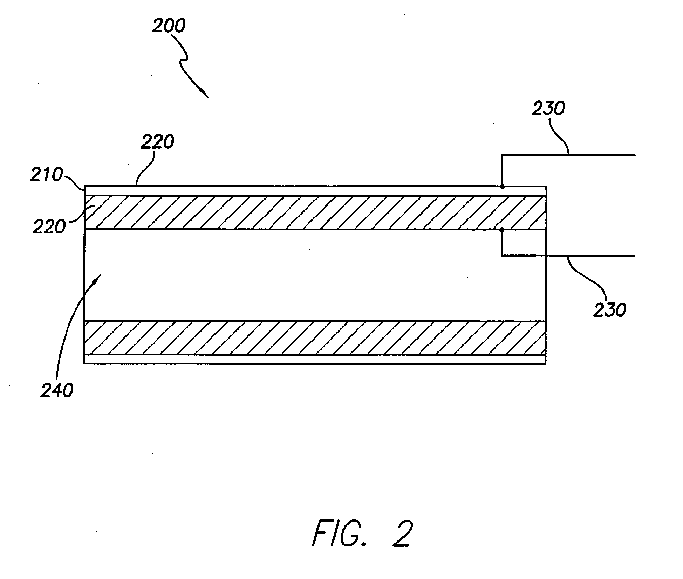

Embodiments of cardiomechanical electric sensors may comprise one or more piezoelectric transducers, which convert mechanical motion into electrical signals. As illustrated in cross section in FIG. 2, in some embodiments, a CMES 200 comprises a tubular and/or annular piezoelectric element 210, either self-supporting or disposed on a supporting structure. In some embodiments, conductors 220 contact the inner and outer surfaces of the tubular or annular element 210. Electrical connections 230 are coupled to the conductors 220.

In preferred embodiments, the sensor 200 is dimensioned for incorporation into a lead. For example, in some embodiments, the outer diameter of the sensor 200 is similar to the outer diameter of a lead, permitting the sensor to be disposed at any position along a lead without causing a profile change that could affect placement of the lead. In some embodiments, one or more of an electrode and/or other sensors is disposed over at least a portion of the sensor 200. A longitudinal passageway 240 through the sensor 200 in the illustrated embodiment permits routing electrical and/or other types of connections there through, for example, from one or more electrodes and/or sensors disposed on the same lead.

The conductors 220 comprise any suitable material known in the art, for example, titanium, titanium alloy, titanium nitride, platinum, platinum alloy, carbon, niobium, niobium alloy, tantalum, tantalum alloy, gold, combinations, and the like. In some embodiments, a patient's tissue is used as one of the conductors. In some embodiments, an elastomer is disposed over the sensor 200 (not illustrated). Preferred elastomers are biocompatible, including, for example, silicones, polyurethanes, ethylene-propylene copolymers, fluorinated elastomers, combinations, and the like.

Preferably, the piezoelectric element comprises a relatively hard material, thereby permitting reliable measurements with only small deflections of the piezoelectric material. Preferred piezoelectric materials are biocompatible, for example, ceramic piezoelectric materials, including ceramic ferroelectric particles, lead zirconate titanate (lead zirconium titanate, PZT), barium titanate, sodium potassium niobate, and the like. In some preferred embodiments, the piezoelectric material comprises $Na_{0.5}K_{0.5}NbO_3$, for example, as described in U.S. Patent No. 6,526,984.

Preferred embodiments of cardiomechanical electric sensors comprise a thin piezoelectric transducer positioned and dimensioned to contact a large surface of myocardial tissue. The large contact area provides more global data than a similar sensor contacting a smaller surface would. Preferred embodiments of CMES detect deformation (strain) and output a voltage that is dependent on the strain. In some preferred embodiments, strain rate-of-change is monitored, which is determined, for example, by taking the first derivative of the voltage signal.

Returning to FIG. 1A, in some embodiments, the sensor system 190 comprises acoustic sensors or sonomicrometers, embodiments of which detect valvular heart sounds directly, for example, as described in U.S. Patent Application No. 11/280,715, titled "METHOD AND APPARATUS FOR MONITORING THE HEART BASED ON CARDIAC ACOUSTICS". The sensor system 190 comprises a combination of sensor types in some embodiments. Furthermore, in some embodiments, one or more of the sensors is integrated with and/or contacts electrical components disposed on the lead, for example, the RV tip electrode 182, RV ring electrode 184, and/or RV coil electrode 186.

In some embodiments, the sensor system 190 comprises at least one sensor located along the more distal region of a lead body, thereby contacting the myocardium, for example, RV tip electrode 182 in FIG. 1A. In other embodiments, the distal region of the lead body may be configured for inserting or embedding into the endocardium. Some embodiments comprise a sensor that contours along a large caliber coronary sinus lead. In some embodiments, at least one sensor contacts the epicardial surface of the right ventricle, for example, if deployed by thoracotomy in a pericardial approach (not illustrated).

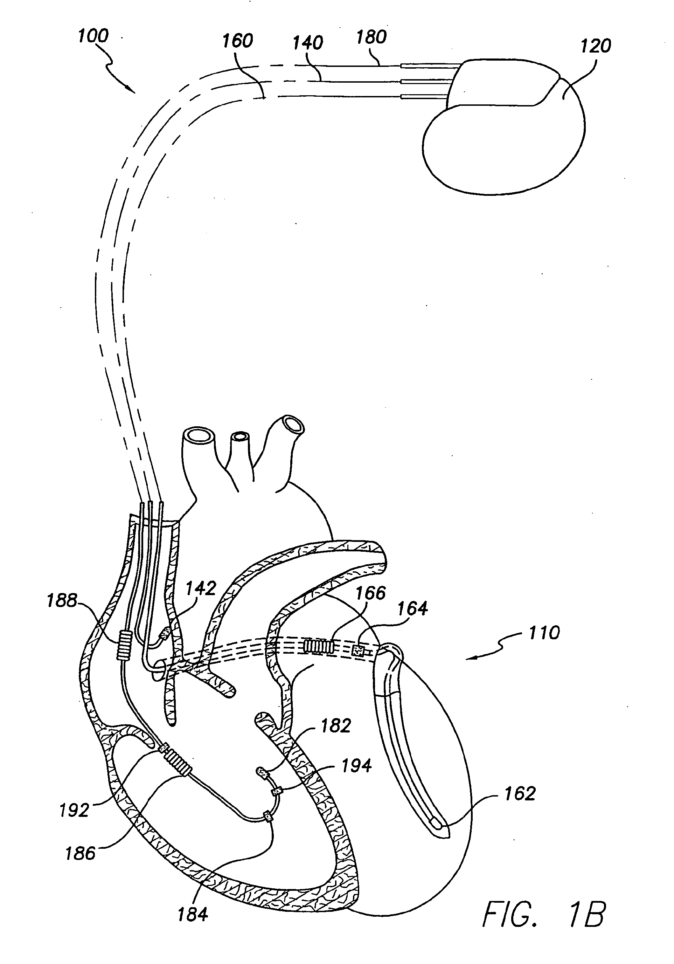

In some embodiments, sensors are positioned to optimize the signal-to-noise ratio (S/N), thereby improving detection of valvular events. Sensor positioning is performed by means known in the art, for example, using ultrasound, visually (e.g., by fiber optic), or other means known in the art. Those skilled in the art will understand that optimal positioning will depend on the particular type of sensor. For example, in the embodiment illustrated in FIG. 1B, in which a first acoustic sensor 192 is disposed on the RV lead 180 proximal to the RV coil 186, the RV lead 180 is adjusted to position the first acoustic sensor 192 near the tricuspid valve. In the illustrated embodiment, a second acoustic sensor 194 is disposed on the RV lead 180 proximal to the RV lead tip electrode/sensor 182, and is positioned near the RV outflow tract (RVOT), just before the pulmonary valve. In some embodiments, acoustic sensor placement at or near the RV apex does not provide as desirable a S/N as the illustrated configuration.

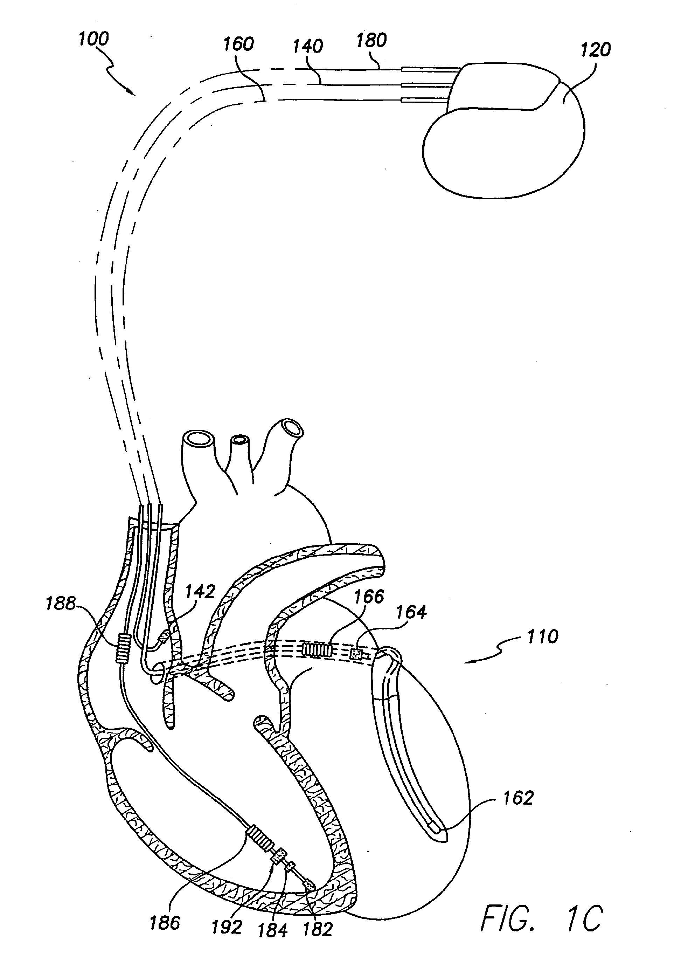

Signal to noise, in some embodiments of accelerometer and/or CMES sensors, is improved by placement at a more basal location because displacement of the basal myocardial region is greater than of apical regions. In the embodiment illustrated in FIG. 1C, the sensor 192 is disposed on the RV lead 180 proximal RV coil 186 and positioned at or near the base of the heart 110, such that the sensor 192 is proximal to and/or contacts the RV free wall. Other embodiments use a separate lead comprising one or more sensors positioned toward the base of the RV free wall. In some embodiments, interference from LV mechanics is reduced by positioning the sensor(s) away from the interventricular septum.

In patients with RV hypokinesia, cardiac acoustic vibrations detected with acoustic sensors (e.g., sonomicrometer or CMES) or impedance-based determinations of valvular events typically provide a better S/N than accelerometer-based technologies because longitudinal displacement of the heart is often impaired in such patients, and consequently, accelerometer-based sensors may provide less optimal signals.

The illustrated implantable stimulation device 100 is capable of treating both fast and slow arrhythmias with stimulation therapy, including cardioversion, defibrillation, and pacing stimulation using a controller system disposed, for example, in the "can," "case," or "case electrode" 120, which in the illustrated embodiment, also serves as return electrode for one or more functions, for example, as disclosed in U.S. Patent Application No. 11/733,083, titled "ELECTROGRAM BASED PHYSIOLOGIC REVERSIBLE RATE RESPONSIVENESS." In the illustrated embodiment, the controller system comprises circuitry and machine readable instructions or logic for treating both fast and slow arrhythmias with stimulation therapy, as well as for determining a pulmonary artery pressure index (PAPi) from the data acquired by the sensor system 190, as discussed in greater detail below. In some embodiments, the PAPi value is an input for the pacing functions of the device 100, and the pacing is modified accordingly. For example, worsening PAPi values may be indicative of worsening left-sided cardiac functioning secondary to interventricular dysynchrony. By modifying the interventricular interval timing interval, left-sided systolic function may be improved. Some embodiments of the controller system further comprises signal processing hardware and/or software known in the art for improving S/N, for example, band pass filters and the like. In some embodiments, the controller system also receives and processes data from a respirometer and/or hypopnea/apnea monitor, for example, for synchronization with a patient's respiration. Accordingly, some embodiments of the device 100 further comprise a respirometer (not illustrated).

Some embodiments of the device 100 further comprise an accelerometer operable to detect patient movement, for example, to determine if the patient is active or resting. Preferably, the accelerometer is installed in or on the can 120.

Moreover, in some embodiments, the apparatus 100 comprises one or more components known in the art for externally monitoring PAPi, for example, by radio frequency (RF) signals, acoustic signals, combinations, and the like. In some preferred embodiments, components implementing these functions are disposed in the can 120. In some embodiments, the apparatus 100 externally outputs at least one of the output of the sensor system 190, a PAPi value, an IVRT value, and the like. The externally output value is monitorable by a user and/or clinician using devices known in the art. In some embodiments, the device 100 outputs a signal indicating a PAPi value detectable by the user without specialized equipment, for example, an audible alarm or tactile signal.

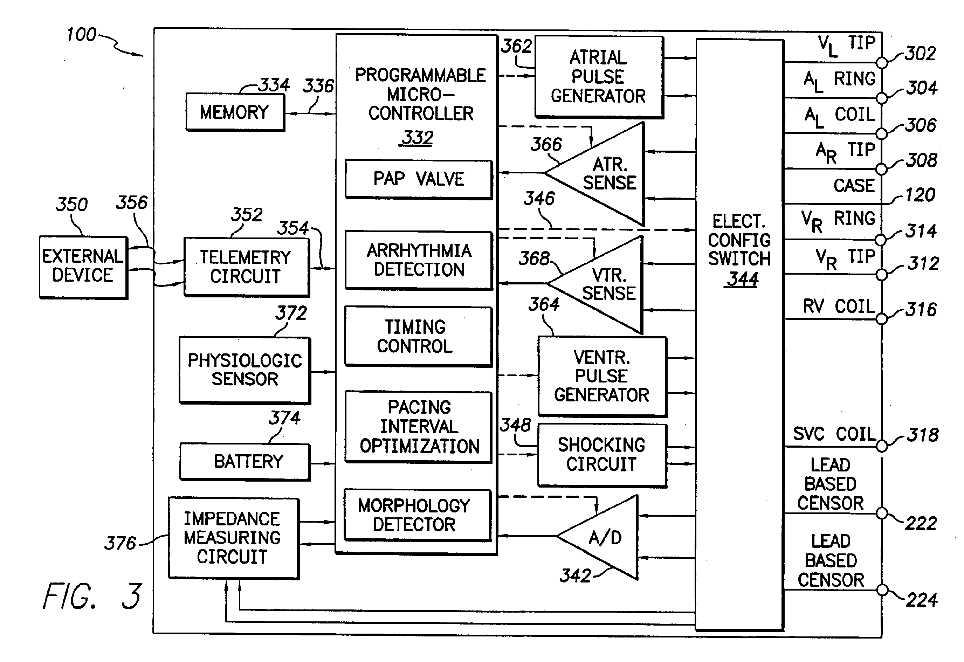

FIG. 3 illustrates a simplified circuit block diagram of an embodiment of the stimulation device 100, which is capable of determining a value representative of right heart pressure (e.g., RVSP, PAP, PAPi, etc.), as well as treating both fast and slow arrhythmias with stimulation therapy, including cardioversion, defibrillation, and pacing stimulation.. Those skilled in the art will understand that the appropriate circuitry could be readily duplicated, eliminated, or disabled in other embodiments to provide a device with a desired combination of features, for example, right heart pressure determination in combination with at least one of cardioversion, defibrillation, and pacing stimulation.

As discussed above, the housing, can, or case 120 may be programmably selected to act as the return electrode for all pacemaker "unipolar" modes. The housing 120 may further be used as a return electrode alone or in combination with one or more of the coil electrodes, 160, 186, and 188, for shocking purposes. The housing 120 further includes a connector (not shown) comprising a plurality of electrode terminals, 302, 304, 306, 308, 310, 312, 314, and 316, (shown schematically and, for convenience, the names of the electrodes to which they are connected are shown next to the terminals), as well as one or more sensor terminals, for example, 322 and 324, to which are coupled the lead-based sensor system 190 for detecting right heart valvular events. For example, in the illustrated embodiment, terminal 322 is coupled to the first acoustic sensor 192, and the terminal 324 is coupled to the second acoustic sensor 194 (FIG. 1 B). Those skilled in the art will understand that more or fewer sensor terminals and/or other types the lead-based sensors are used in other embodiments.

To achieve left chamber sensing, pacing and shocking, the connector includes at least a left ventricular tip terminal ($V_L$ TIP) 302, a left atrial ring terminal ($A_L$ RING) 304, and a left atrial shocking terminal ($A_L$ COIL) 306, which are adapted for connection to the left ventricular tip electrode 162, the left atrial ring electrode 164, and the left atrial coil electrode 166, respectively.

To support right chamber sensing, pacing and shocking, the connector further includes a right ventricular tip terminal ($V_R$ TIP) 312, a right ventricular ring terminal ($V_R$ RING) 314, a right ventricular shocking terminal ($R_V$ COIL) 316, and an SVC shocking terminal (SVC COIL) 318, which are adapted for connection to the right ventricular tip electrode 182, right ventricular ring electrode 184, the RV coil electrode 186, and the SVC coil electrode 188, respectively.

At the core of the device 100 is at least one programmable microcontroller 332, which determines a right heart pressure value from the output of the sensor system 190, as well as controlling the various modes of stimulation therapy. As is well known in the art, the microcontroller 332 typically includes a microprocessor, or equivalent control circuitry, designed specifically for controlling the delivery of stimulation therapy and may further include memory (for example, RAM or ROM memory), logic and timing circuitry, state machine circuitry, I/O circuitry, and the like. Typically, the microcontroller 332 includes the ability to process or monitor input signals (data) as controlled by a program code stored in a designated block of memory. Any suitable microcontroller 332 may be used that carries out the functions described herein.

Signals from the housing 120, electrode terminals, and sensor terminals comprise inputs of an analog-to-digital (A/D) data acquisition system 342, an output of which is coupled to the microcontroller 332. The data acquisition system 342 is configured to acquire signals from the sensor system 190, intracardiac electrogram (IEGM) signals, convert analog signals into digital signals, and/or store the digital signals for later processing and/or telemetric transmission to an external device 350. The data acquisition system 342 is coupled to the housing 120, right atrial lead 140, the coronary sinus lead 160, and the right ventricular lead 180 through a switch 344, permitting sampling of cardiac signals across any pair of electrodes as desired.

The microcontroller 332 is further coupled to a memory 334 by a suitable data/address bus 336, wherein data and programmable operating parameters used by the microcontroller 332 are stored and modified, as required, in order to customize the operation of the device 100 to suit the needs of a particular patient. Such operating parameters define, for example, frequency of right heart pressure determination, pacing pulse amplitude, pulse duration, electrode polarity, rate, sensitivity, automatic features, arrhythmia detection criteria, and the amplitude, waveshape, and vector of each shocking pulse to be delivered to the patient's heart 110 within each respective tier of therapy.

Advantageously, the operating parameters of the implantable device 100 may be non-invasively programmed into the memory 334 through a telemetry circuit 352 in telemetric communication with the external device 350, such as a programmer, transtelephonic transceiver, or a diagnostic system analyzer. The telemetry circuit 352 is activated by the microcontroller by a control signal 354. The telemetry circuit 352 advantageously allows right heart pressure, IEGMs, and status information relating to the operation of the device 100 (as contained in the microcontroller 332 or memory 334) to be sent to the external device 350 through an established communication link 356.

In the illustrated embodiment, analog signals encoding right heart valvular events from the sensor network 190 are directed by the switch 344 to the data acquisition system 342 where they converted into digital signals. From these digital signals, the microcontroller 232 executes instructions that provide a value indicative of a right heart pressure and/or PAP, for example, PAPi, as discussed in greater detail below.

In the illustrated embodiment, an atrial pulse generator 362 and a ventricular pulse generator 364 generate pacing stimulation pulses for delivery by the right atrial lead 140, the right ventricular lead 180, and/or the coronary sinus lead 160 via the electrode configuration switch 344. It is understood that in order to provide stimulation therapy in each of the four chambers of the heart, the atrial and ventricular pulse generators, 362 and 364, may include dedicated, independent pulse generators, multiplexed pulse generators, or shared pulse generators. The pulse generators, 362 and 364, are controlled by the microcontroller 332 via appropriate control signals to trigger or inhibit the stimulation pulses.

The microcontroller 332 further includes timing control circuitry, which controls the timing of such stimulation pulses (e.g., pacing rate, atrio-ventricular (AV) delay, atrial interconduction (A-A) delay, ventricular interconduction (V-V) delay, etc.) as well as to keep track of the timing of refractory periods, post-ventricular atrial refractory period (PVARP) intervals, noise detection windows, evoked response windows, alert intervals, marker channel timing, etc.

The switch 344 includes a plurality of switches for connecting the desired electrodes to the appropriate I/O circuits, thereby providing complete electrode programmability. Accordingly, the switch 344, in response to a control signal 346 from the microcontroller 332, determines the polarity of the stimulation pulses (e.g., unipolar, bipolar, combipolar, etc.) by selectively closing the appropriate combination of switches (not shown) as is known in the art. In this embodiment, the switch 344 also supports simultaneous high resolution impedance measurements, such as between the case or housing 120, the right atrial electrode 142, and right ventricular electrodes 182 and 184, as described in greater detail below.

Atrial sensing circuits 366 and ventricular sensing circuits 368 may also be selectively coupled to the right atrial lead 140, coronary sinus lead 160, and the right ventricular lead 180, through the switch 344 for detecting the presence of cardiac activity in each of the four chambers of the heart. Accordingly, the atrial (ATR.Sense) and ventricular (VTR.Sense) sensing circuits, 366 and 368, may include dedicated sense amplifiers, multiplexed amplifiers, or shared amplifiers. The switch 344 determines the "sensing polarity" of the cardiac signal by selectively closing the appropriate switches, as is also known in the art. In this way, the clinician may program the sensing polarity independently of the stimulation polarity.

Each sensing circuit 366 and 368 preferably employs one or more low power, precision amplifiers with programmable gain and/or automatic gain control, bandpass filtering, and a threshold detection circuit, as known in the art, to selectively sense the cardiac signal of interest. The automatic gain control enables the device 100 to deal effectively with the difficult problem of sensing the low amplitude signal characteristics of atrial or ventricular fibrillation. The outputs of the atrial and ventricular sensing circuits 366 and 368 are connected to the microcontroller 332 which, in turn, are able to trigger or inhibit the atrial and ventricular pulse generators 362 and 364, respectively, in a demand fashion in response to the absence or presence of cardiac activity in the appropriate chambers of the heart.

For arrhythmia detection, the device 100 utilizes the atrial and ventricular sensing circuits 366 and 368 to sense cardiac signals to determine whether a rhythm is physiologic or pathologic. As used herein "sensing" is reserved for the noting of an electrical signal, and "detection" is the processing of these sensed signals and noting the presence of an arrhythmia. The timing intervals between sensed events (e.g., P-waves, R-waves, and depolarization signals associated with fibrillation) are then classified by the microcontroller 332 by comparing them to a predefined rate zone limit (i.e., bradycardia, normal, low rate ventricular tachycardia, high rate ventricular tachycardia, and fibrillation rate zones) and various other characteristics (e.g., sudden onset, stability, physiologic sensors, and morphology, etc.) in order to determine the type of remedial therapy that is needed (e.g., bradycardia pacing, anti-tachycardia pacing, cardioversion shocks or defibrillation shocks, collectively referred to as "tiered therapy").

In a preferred embodiment, the device 100 further includes one or more physiologic sensor 372, commonly referred to as a "rate-responsive" sensor because it is typically used to adjust the pacing stimulation rate according to the activity state of the patient. The activity state of a patient reflect the physical activity level of a patient and/or the emotional state of the patient as caused by sympathetic nervous system activation, as measured by physiological sensor 372. The physiological sensor 372 may be a mechanical sensor that measures breathing rate or a pressure sensor that measure changes in intrathoracic pressures as influenced by inspiration. Other physiological sensors, for example, may be used to detect changes in cardiac output or changes in the physiological condition of the heart including myocardial contractility states. As discussed above, in some embodiments, the physiological sensor comprises an accelerometer, which is useful for determining patient motion or diurnal changes in activity (e.g., detecting sleep and wake states). In some embodiments, the physiologic sensor 372 may be a multi-modal composite of different physiologic sensors used to determine a pacing rate control parameter. Accordingly, the microcontroller 332 responds by adjusting the various pacing parameters (such as rate, AV Delay, V-V Delay, etc.) at which the atrial and ventricular pulse generators 362 and 364 generate stimulation pulses.

The stimulation device additionally includes a battery 374 which provides operating power to all of the circuits shown in FIG. 3. For the device 100, which employs shocking therapy, the battery 374 is capable of operating at low current drains for long periods of time and then be capable of providing high-current pulses (for capacitor charging) when the patient requires a shock pulse. The battery 374 must also have a predictable discharge characteristic so that elective replacement time can be detected. Accordingly, embodiments of the device 100 including shocking capability preferably employ lithium/silver vanadium oxide batteries. For embodiments of the device 100 not including shocking capability, the battery 374 will preferably be lithium iodide, carbon monoflouride, a hybrid of the two, or any other suitable battery.

As further shown in FIG. 3, the device 100 is shown as having an impedance measuring circuit 376 which is enabled by the microcontroller 332 via a control signal.

In the case where the device 100 is intended to operate as an implantable cardioversion/defibrillator (ICD) device, it must detect the occurrence of an arrhythmia, and automatically apply an appropriate electrical shock therapy to the heart aimed at terminating the detected arrhythmia. To this end, the microcontroller 332 further controls a shocking circuit 348 by way of a control signal. The shocking circuit 348 generates shocking pulses of low (up to about 0.5 joules), moderate (about 0.5-10 joules), or high energy (about 11-40 joules), as controlled by the microcontroller 332. Such shocking pulses are applied to the patient's heart 110 through at least two shocking electrodes, and as shown in this embodiment, selected from the left atrial coil electrode 160, the RV coil electrode 186, and/or the SVC coil electrode 188. As noted above, the housing 120 may act as an active electrode in combination with the RV electrode 186, or as part of a split electrical vector using the SVC coil electrode 188 or the left atrial coil electrode 166 (i.e., using the RV electrode as a common electrode).

Cardioversion shocks are generally considered to be of low to moderate energy level (so as to minimize pain felt by the patient), and/or synchronized with an R-wave and/or pertaining to the treatment of tachycardia. Defibrillation shocks are generally of moderate to high energy level (i.e., corresponding to thresholds in the range of about 5-40 joules), delivered asynchronously (since R-waves may be too disorganized), and pertaining exclusively to the treatment of fibrillation. Accordingly, the microcontroller 332 is capable of controlling the synchronous or asynchronous delivery of the shocking pulses.

As mentioned previously, the timing control circuitry is used to control the timing of such stimulation pulses. Various methodologies are available for providing the timing of pacing impulses in such systems. In a normal heart, increased activity will cause increases in heart rate due to activation of the sympathetic nervous system. The elevated heart rate typically results in increased cardiac output because the stroke volume of blood per heart beat is generally maintained as the heart rate increased. Thus in normal hearts without disease, these increased rate responses associated with increased physical activity or stress situations are generally appropriate and beneficial. To mimic this normal physiologic response in a CRT device, the CRT device may be programmed with a positive rate response algorithm that uses a physiologic sensor to detect changes associated with increased activity, such as in increase in movement or breathing rate. The activity or workload detection may be performed using any of a variety of mechanical sensors known in the art. Upon detection of the increased activity, the pacing rate is increased to mimic the physiologic heart rate response to stress or activity. The AV/PV (atrial stimulation pulse to ventricular stimulation pulse or intrinsic P-wave to ventricular stimulation pulse, respectively) interval may also be increased along with the increased heart rate.

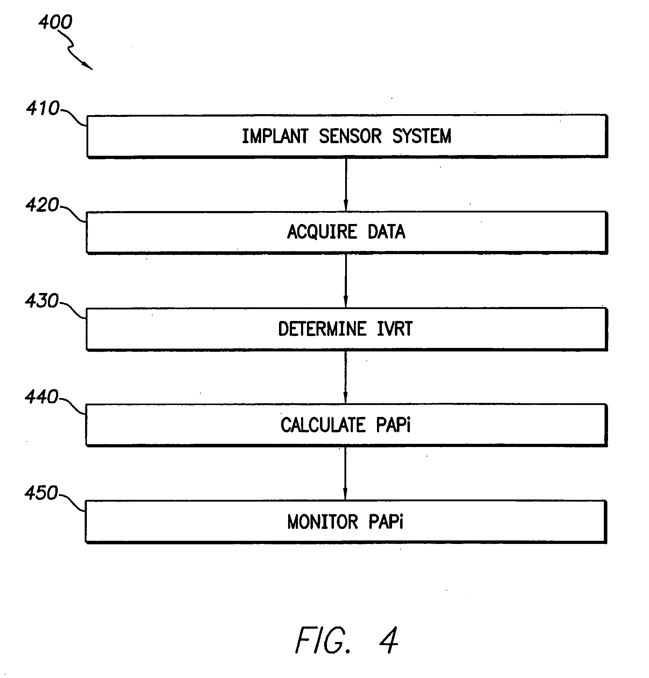

An embodiment of a method 400 for determining a systolic pulmonary arterial pressure index (PAPi) is illustrated in FIG. 4, and described with reference to the embodiments of the device illustrated in FIG. 1A. In step 410, the sensor system 190 is implanted in a patient and the sensors positioned as discussed above. In step 420, data indicative of or encoding right heart valvular events is acquired using the sensor system. In step 430, an RV isovolumetric relaxation time (IVRT) is determined from the valvular event data. In step 440, a pulmonary artery pressure index (PAPi) is calculated from the IVRT value. In step 450, the PAPi is monitored over time.

In step 420, data indicative of or encoding timing of right heart valvular events is acquired using the sensor system 190, including at least pulmonary valve closure (PVc) and tricuspid valve opening (TVo). PVc corresponds to the end of mechanical systole (ES) and TVo corresponds to the onset of mechanical diastole (D). In some embodiments, the valvular events are detected directly, for example, when using some embodiments of CMES, acoustic, and/or sonomicrometer sensors.

In other embodiments, the valvular events are detected indirectly. For example, the output of some sensor types, such as some embodiments of CMES, accelerometer, and/or electrical impedance sensors, may be used to generate waveform data that may be used to derive the timing valvular events of interest. Those skilled in the art will understand that the particular method used to determine the timing of the events depends on the particular type of sensor used. Because accelerometers and CMES detect pressure waves resulting from myocardial motion, for example, the motion of the RV free wall, valvular events are determined by analyzing the pressure waveforms, for example, by determining the time difference between changes in slope signifying the end of mechanical systole (ES) and onset of mechanical diastole (D). Techniques for identifying fiducial points on acquired waveforms are known to those skilled in the art. In some embodiments using electrical impedance sensors, notches in dynamic impedance waveforms acquired between electrodes in the right heart can be used to determine time of valvular events as well, for example, as discussed in connection with left heart valvular events (e.g., aortic valve opening and closing) in U.S. Patent No. 7,065,400 entitled "METHOD AND APPARATUS FOR AUTOMATICALLY PROGRAMMING CRT DEVICES".

Some embodiments use a combination of methods for determining valvular events. Because accelerometer and/or CMES transducers indirectly detect the effect of valvular events through their effects the motion of an RV free wall, some embodiments of these methods include a time lag or delay t between an actual valvular event and its effect on the RV free wall. Some embodiments of methods for determining valvular events using accelerometer/CMES sensors in conjunction with direct detection of valvular events include a correction for the time lag t.

In some embodiments, S/N of the valvular event data is improved using signal processing hardware and/or software known in the art, for example, by ensemble averaging of multiple waveforms. Signal to noise is also improved in some embodiments by acquiring and/or using valvular event data from times of relative apnea, for example, at the end of expiration using a respirometer and/or hypopnea/apnea monitor to determine periods of relative apnea. In some embodiments S/N is improved by using data collected at times of rest, for example, as determined using motion sensors or accelerometers, for example, disposed on the can 140 as discussed above. Combinations of these methods are also used in some embodiments.

In step 430, an RV isovolumetric relaxation time (IVRT) is determined from the valvular event data acquired in step 420. IVRT is the time between closing of the pulmonary valve and opening of the tricuspid valve, and is directly correlated with systolic PAP and inversely correlated with heart rate. That is, the greater the RV pressure is when the pulmonary valve closes, the longer it takes for the RVSP to decrease to the level of right atrial pressure, assuming a constant RV isovolumic relaxation rate.

The relationship between IVRT and PAP is described, for example, in L. Burstin "Determination of pressure in the pulmonary artery by external graphic recordings" Br. Heart J. 1967, 29:396-404 in which phonocardiography and pulse tracings were used to determine IVRT. These results have been validated in a number of studies, including: V. Dambrauskaite, et al. "The Evaluation of Pulmonary Hypertension Using Right Ventricular Myocardial Isovolumic Relaxation Time" J. Am. Soc. Echo. 2005, 18: 1113-20 (correlating IVRT from tissue Doppler measurements of RV free wall segments to invasively measured pulmonary artery pressures); P. Caso, et al. "Association between myocardial right ventricular relaxation time and pulmonary arterial pressure in chronic obstructive lung disease analysis by pulsed Doppler tissue imaging" J. Am. Echo. 2001, 14:970-77 (correlating PAP and PAPi with tricuspid valve longitudinal motion measured by Doppler echocardiogram); and L. Hatle & G.R. Sutherland "Regional myocardial function - a new approach" Eur. Heart J. 2000, 21:1337-57 (correlating PAP with regional RV IVRT derived from RV myocardial wall motion. IVRT has not been previously determined using lead-based sensors designed for detecting right heart mechanics and valvular events, however.

In step 440, a unitless pulmonary artery pressure index (PAPi) is calculated from the IVRT value according to Eq. 1, where "RR" is the duration of the cardiac cycle. In some embodiments, the RR interval is calculated using intracardiac electrograms (EGM), for example, as described in H.C. Bazett "An analysis of the time relationship of electrocardiograms" Heart 1920, 7:353-70.

$$PAPi = \frac{IVRT}{RR^{\frac{1}{2}}} \qquad\qquad Eq.\ 1$$

The linear relationship between PAPi and IVRT', a tissue Doppler-derived IVRT, was used to measure systolic and mean PAP in V Dambrauskaite, et. al, "The Evaluation of Pulmonary Hypertension Using Right Ventricular Myocardial Isovolumic Relaxation Time" J. Am. Soc. Echo. 2005, 18: 1113-20. A correlation between PAPi and pulmonary artery systolic pressure is described in P. Caso, et al. "Association between myocardial right ventricular relaxation time and pulmonary arterial pressure in chronic obstructive lung disease analysis by pulsed Doppler tissue imaging" J. Am. Echo. 2001, 14:970-77.

In step 450, PAPi is monitored for chronic assessment using trend analysis as is known in the art. In some embodiments, an alarm or alert is triggered if significant changes in PAPi are detected acutely.

For example, in some embodiments, chronic PAPi is determined by averaging PAPi over a selected time period on the order of months, for example, from about 1 to about 6 months. The time period may be preprogrammed or user selectable. Acute PAPi is then similarly determined by averaging PAPi over a period of days, for example, from about 1 to about 7 days. Eq. 2 provides an embodiment of a method for calculating an IVRT PAP index (IPI).

$$IPI = \frac{\text{acute PAPi}}{\text{chronic PAPi}} \qquad\qquad Eq.\ 2$$

IPI approaches unity under stable conditions. Significant changes in IPI, for example, IPI values of greater than about 1.2, greater than about 1.5, or greater than about 2 or about 3, may indicate the need for diagnostic testing and/or changes in therapy.

EXAMPLE 1

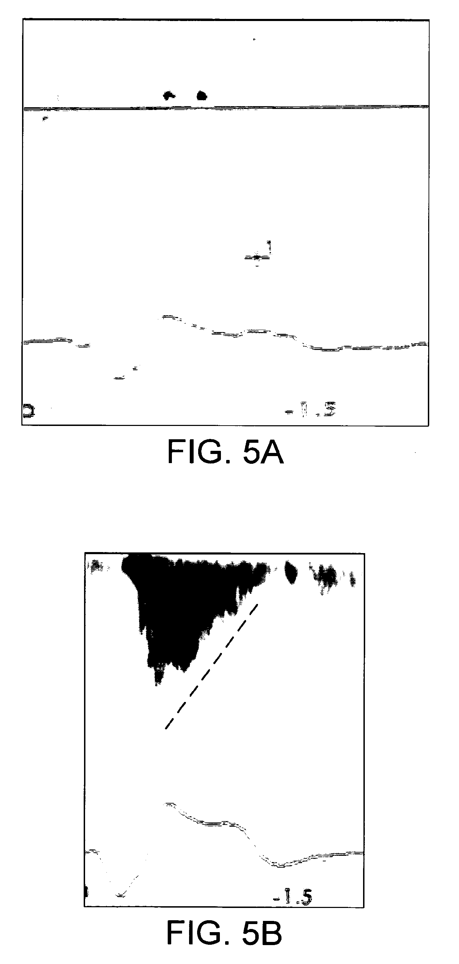

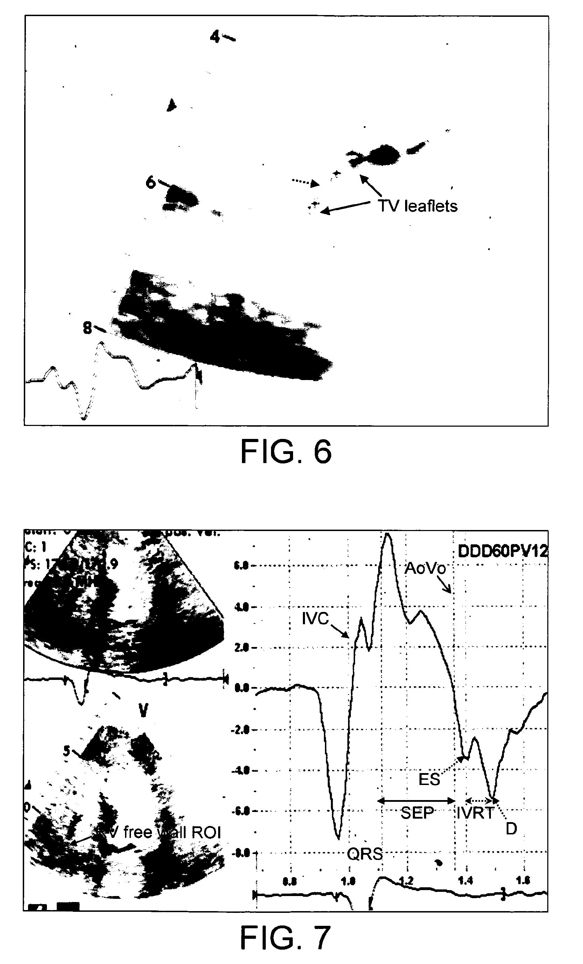

This example illustrates some limitations of echocardiograhic detection of PHT, which depends on the presence of significant tricuspid regurgitation (TR). The detection of maximal TR flow may be used to calculate the pressure gradient between the right ventricle and right atrium. By adding the pressure gradient to the right atrial pressure, the right ventricular systolic pressure may be estimated. Similarly, pulmonary artery diastolic pressure (PADP) may be estimated by calculating the pressure gradient between the right ventricle and right atrium at the time of pulmonary artery valve opening, since right ventricular and pulmonary artery pressure has been shown to equilibrate at this point in the cardiac cycle. PADP in turn may be used to estimate left ventricular filling pressure. B. Stephan, et al., "Noninvasive Estimation of Pulmonary Artery Diastolic Pressure in Patients with Tricuspid Regurgitation by Doppler Echocardiography" Chest, 1999, 116; 73-77. FIG. 5A illustrates a normal echocardiogram waveform suitable for quantifying peak blood cell velocities within a TR jet according to a modified Bernoulli equation. RV systolic pressure is then derived, for example, as described in T. Skjaerpe & L. Hatle "Diagnosis and assessment of tricuspid regurgitation with Doppler ultrasound" Martinus Nijhoff:The Hague, 1981, 299-304. FIG. 5B is an echocardiogram of a patient exhibiting inadequate regurgitant tricuspid blood flow to permit quantifying peak blood cell velocities, as indicated by the faint TR spectral envelope. Without the identification of the TR jet, calculation of RVSP or PADP is problematic. Structural changes of the tricuspid valve may also prevent RVSP from being accurately estimated with conventional echo Doppler techniques, for example, as illustrated in FIG. 6, which is an echocardiogram of a tricuspid valve with poor coaptation. The TV leaflets are indicated with solid arrows, and the opening between the valve leaflets, as indicated by the broken arrow, indicative of dilation of the tricuspid valve annulus.

EXAMPLE 2

In this example, RV free wall velocities at a region of interest are acquired by tissue Doppler recordings, and the corresponding events identified. FIG. 7 illustrates a myocardial velocity-time graph of a region of interest (ROI) on an RV free wall. Velocity data were acquired by tissue Doppler recordings. IVRT (dashed horizontal double headed arrow) was calculated by measuring the time interval between the end of the systolic wave (ES) and the beginning of diastolic wave (D). The systolic ejection phase (SEP) of the left heart occurs over the period indicated by a double headed arrow. At the end of SEP, the aortic valve closes (AoVc). Pulmonary valve closure occurs at about the same time as AoVc, followed by tricuspid valve opening (TVo). Motion of the RV free wall corresponding to each of these events of the cardiac cycle is not simultaneous with the event because the motion generated by the events is transmitted through cardiac tissue and intracavity chamber before they are detected at the ROI, thereby resulting in a time lag, as discussed above. IVC is the isovolumic contraction, which occurs at about the time of electrical systole (QRS).

EXAMPLE 3

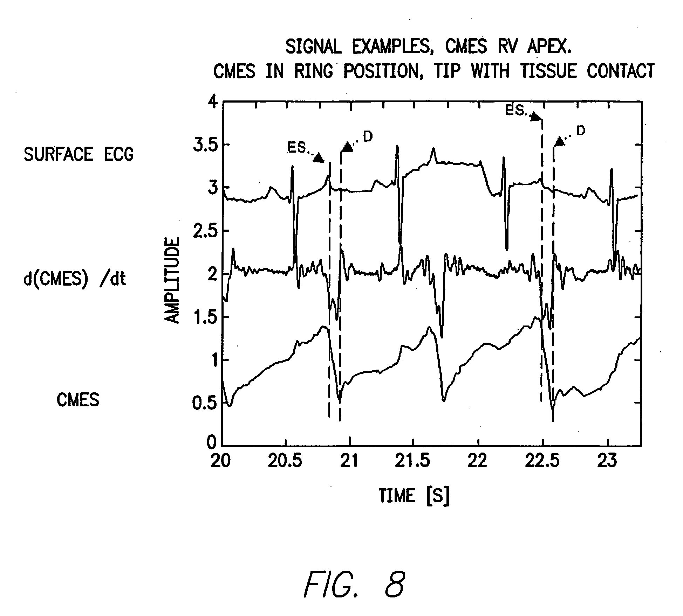

FIG. 8 compares waveforms generated by surface ECG and implanted lead-based cardiomechanical sensor (CMES) measurements. In this experiment, the CMES sensor was positioned at the RV apex, in the ring position, with the lead tip contacting the myocardium. The top trace is the ECG; the middle trace is the first derivative of CMES output (d(CMES)/dt); and the bottom trace is the CMES output. "ES" indicates the regional end-systole, and "D" indicates the onset of regional mechanical diastole. Three cardiac cycles are illustrated, indicating the consistency of the signals. Note the similarities between the d(CMES)/dt trace and the ultrasound trace illustrated in FIG. 7.

EXAMPLE 4

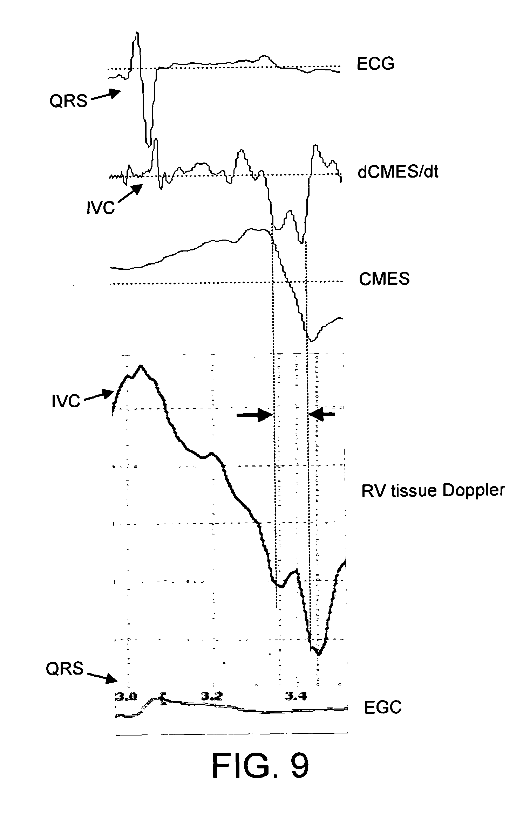

FIG. 9 illustrates the similarities between ECG, implanted lead-based CMES, and tissue Doppler measurements. CMES and tissue Doppler are of the RV free basal wall. The end of regional mechanical systole (ES) and onset of mechanical diastole (D) are easily located in the CMES waveform. Again, similarities between the d(CMES)/dt trace and tissue Doppler trace are apparent.

EXAMPLE 5

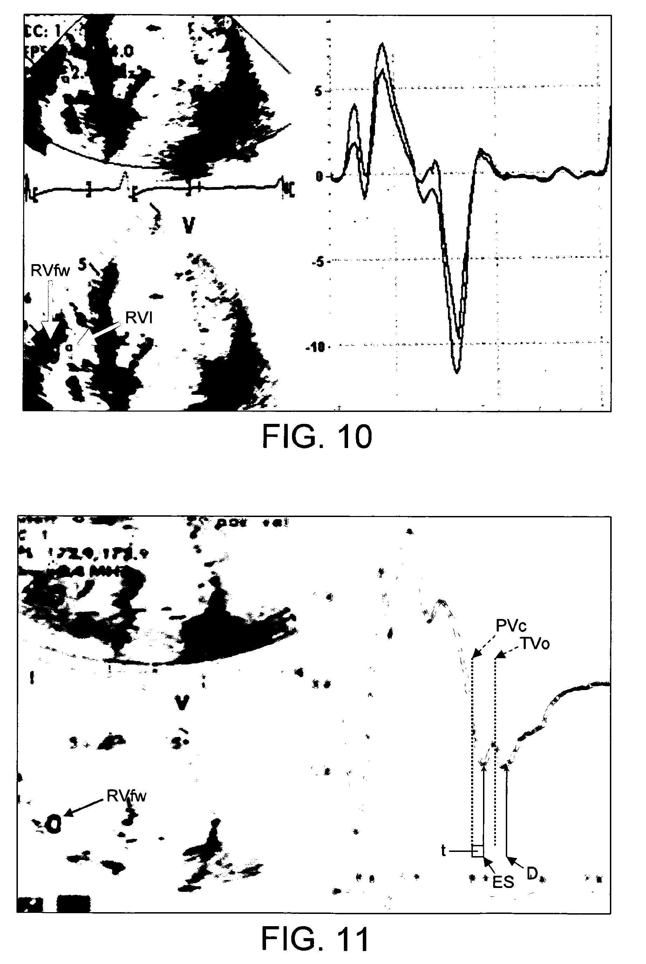

The motion of the RV myocardium imparted on the RV lead and tethers along the lead is similar to the Doppler derived waveforms recorded from the RV free wall. In FIG. 10, the tissue-Doppler-measured velocity-time curves of an RV lead (RVl) and a region of interest on the RV free wall (RVfw) are overlaid, illustrating the similarities in the velocity-time graph.

EXAMPLE 6

An example of a time lag t between an actual valvular event and its effect on myocardial motion is illustrated in FIG. 11. The timing of the pulmonary valve closure (PVc) and tricuspid valve opening (TVo) were determined by echocardiography (dashed lines). The timing of the end of systole (ES) and onset of diastole (D) were determined using a CMES sensor (solid lines). The time lag t is indicated by the box between PVc and ES.

Those skilled in the art will understand that the disclosed subject matter may be embodied in other specific forms without departing from its essential characteristics. The described embodiments are to be considered in all respects only as illustrative and not restrictive, and the scope of the disclosure is, therefore, indicated by the appended claims rather than by the foregoing description. Those skilled in the art will also understand that, in some embodiments, the steps of the methods need not be performed sequentially and/or in the order described. All changes that come within the meaning and range of equivalency of the claims are to be embraced within their scope.

**Claims**

**1.** An implantable cardiac stimulation device comprising:

at least one lead adapted to be implanted within the right ventricle of the heart, wherein the at least one lead is adapted to provide therapeutic stimulation to the heart of the patient;
at least one valve sensor wherein the sensor is adapted to be implanted within a chamber of the heart wherein the at least one sensor is further adapted to sense valvular events within the right ventricle of the heart and provide valvular event signals indicative thereof;
a controller that induces the delivery of therapeutic stimulation to the heart of the patient via the at least one lead and receives the valvular event signals from the at least one valve sensor, wherein the controller uses the valvular event signals to periodically determine a time parameter at least partially representative of the time period between the closing of the pulmonary valve and the opening of the tricuspid valve of the right ventricle of the heart and wherein the controller assess whether the periodically determined time parameter is indicative of increasing pulmonary artery pressure.

**2.** The device of claim 1, wherein the at least one valve sensor is a pressure sensor, which is preferably a CMES sensor, that senses a signal indicative of the movement of the walls of the right ventricle or wherein the at least one valve sensor comprises an impedance sensor that senses the impedance within the right ventricle to determine the occurrence of the valvular events or wherein the at least one sensor comprises an accelerometer.

**3.** The device of claim 1, wherein the controller assess whether the periodically determined time parameter is indicative of increasing pulmonary artery pressure by normalizing the time characteristic.

**4.** The device of claim 3, wherein at least one lead also provides an intra-cardiac electrogram signal to the controller and wherein the time an intra-cardiac electrogram signal to the controller and wherein the time parameter is normalized by dividing the time characteristic by the square root of a measured time period for a cycle of the intra-cardiac electrogram signal so as to correct the time parameter for heart rate.

**5.** The device of claim 1, wherein the controller uses the normalized time parameter to periodically assess whether the normalized time parameter is potentially indicative of increasing pulmonary artery pressure.

**6.** The device of claim 1, wherein the controller determines whether the periodically determined time parameter is potentially indicative of increasing pulmonary artery pressure by periodically averaging a first time period set of normalized time parameter values and a second time period set of normalized time parameter values wherein the second time period a longer duration than the first time period and then determining a ratio between the first time period set of normalized time parameter values and the second time period set of normalized time parameter values.

7. A method for determining a pulmonary artery pressure index comprising:

    positioning a lead comprising a sensor system at a right ventricle;
    acquiring with the sensor system data encoding right heart valvular timing, wherein the data does not comprise direct pressure measurement;
    extracting from the data encoding right heart valvular timing a pulmonary valve closure and a tricuspid valve opening;
    determining an isovolumetric relaxation time from the pulmonary valve closure and the tricuspid valve opening; and
    calculating a pulmonary artery pressure index from the isovolumetric relaxation time.

8. The method of claim 7, wherein the sensor system comprises a plurality of right ventricular electrodes for acquiring real-time impedance waveforms or an acoustic sensor for acquiring valvular heart sounds or an

9. The method of claim 7, further comprising applying a band-pass filter to the data acquired by the sensor system to improve the signal to noise ratio.

10. The method of claim 7, wherein at least a portion of the data is acquired during a period of rest.

11. The method of claim 8, further comprising monitoring pulmonary artery pressure index over time to detect acute changes.

12. A device for measuring a pulmonary artery pressure index comprising:

    an implantable lead comprising a sensor system operable to detect right-heart valvular timing and output a corresponding signal, wherein the sensor system does not comprise an electrical sensor or a direct pressure measuring sensor; and
    an implantable controller system coupled to the sensor system operable to convert the signal from the sensor system into a pulmonary artery pressure.

13. The device of claim 12, wherein the controller system is disposed in a case.

14. The device of claim 12, further comprising a respirometer coupled to the controller system.

15. The device of claim 12, further comprising a left atrial lead and a coronary sinus lead, both of which are coupled to the controller system, wherein the implantable lead is a right ventricular lead, and
    the controller is operable to treat cardiac arrhythmia with stimulation therapy.

FIG. 1A

FIG. 1B

FIG. 1C

FIG. 2

FIG. 3

400

410 — IMPLANT SENSOR SYSTEM

420 — ACQUIRE DATA

430 — DETERMINE IVRT

440 — CALCULATE PAPi

450 — MONITOR PAPi

FIG. 4

FIG. 5A

FIG. 5B

FIG. 6

FIG. 7

SIGNAL EXAMPLES, CMES RV APEX.
CMES IN RING POSITION, TIP WITH TISSUE CONTACT

FIG. 8

FIG. 9

FIG. 10

FIG. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6886411 B
- US 6526984 B
- US 280715 A
- US 733083 A
- US 7065400 B

### Non-patent literature cited in the description

- **L. BURSTIN.** Determination of pressure in the pulmonary artery by external graphic recordings. *Br. Heart J.,* 1967, vol. 29, 396-404
- **V. DAMBRAUSKAITE et al.** The Evaluation of Pulmonary Hypertension Using Right Ventricular Myocardial Isovolumic Relaxation Time. *J. Am. Soc. Echo.,* 2005, vol. 18, 1113-20
- **P. CASO et al.** Association between myocardial right ventricular relaxation time and pulmonary arterial pressure in chronic obstructive lung disease analysis by pulsed Doppler tissue imaging. *J. Am. Echo.,* 2001, vol. 14, 970-77
- **L. HATLE ; G.R. SUTHERLAND.** Regional myocardial function - a new approach. *Eur. Heart J.,* 2000, vol. 21, 1337-57
- **H.C. BAZETT.** An analysis of the time relationship of electrocardiograms. *Heart,* 1920, vol. 7, 353-70
- **V DAMBRAUSKAITE.** The Evaluation of Pulmonary Hypertension Using Right Ventricular Myocardial Isovolumic Relaxation Time. *J. Am. Soc. Echo.,* 2005, vol. 18, 1113-20
- **B. STEPHAN et al.** Noninvasive Estimation of Pulmonary Artery Diastolic Pressure in Patients with Tricuspid Regurgitation by Doppler Echocardiography. *Chest,* 1999, vol. 116, 73-77
- **T. SKJAERPE ; L. HATLE.** Diagnosis and assessment of tricuspid regurgitation with Doppler ultrasound. *Martinus Nijhoff:The Hague,* 1981, 299-304